(19) Europäisches Patentamt
European Patent Office
Office européen des brevets

(11) Veröffentlichungsnummer: **0 338 424 B1**

# (12) EUROPÄISCHE PATENTSCHRIFT

(45) Veröffentlichungstag der Patentschrift: **24.11.93**

(51) Int. Cl.⁵: **C07D 239/42**, A01N 47/36

(21) Anmeldenummer: **89106595.5**

(22) Anmeldetag: **13.04.89**

Die Akte enthält technische Angaben, die nach dem Eingang der Anmeldung eingereicht wurden und die nicht in dieser Patentschrift enthalten sind.

## (54) N-((6-Trifluormethylpyrimidin-2-yl)-aminocarbonyl)-2-carboalkoxy-benzolsulfonamide, Verfahren zu ihrer Herstellung und ihre Verwendung.

(30) Priorität: **22.04.88 DE 3813623**

(43) Veröffentlichungstag der Anmeldung:
**25.10.89 Patentblatt 89/43**

(45) Bekanntmachung des Hinweises auf die Patenterteilung:
**24.11.93 Patentblatt 93/47**

(84) Benannte Vertragsstaaten:
**CH DE FR GB IT LI**

(56) Entgegenhaltungen:
**EP-A- 0 007 687**
**EP-A- 0 084 020**
**US-A- 4 563 211**

(73) Patentinhaber: **BASF Aktiengesellschaft**
**Carl-Bosch-Strasse 38**
**D-67063 Ludwigshafen(DE)**

(72) Erfinder: **Westphalen, Karl-Otto, Dr.**
**Mausbergweg 58**
**D-6720 Speyer(DE)**
Erfinder: **Hamprecht, Gerhard, Dr.**
**Rote-Turm-Strasse 28**
**D-6940 Weinheim(DE)**
Erfinder: **Würzer, Bruno, Dr.**
**Rüdigerstrasse 13**
**D-6701 Otterstadt(DE)**
Erfinder: **Meyer, Norbert, Dr.**
**Dossenheimer Weg 22**
**D-6802 Ladenburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 338 424 B1

## Beschreibung

Die vorliegende Erfindung betrifft N-((6-Trifluormethylpyrimidin-2-yl)aminocarbonyl)-2-carboalkoxybenzolsulfonamide der allgemeinen Formel Ia,

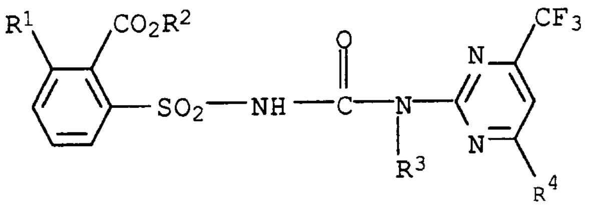

Ia

in der die Substituenten folgende Bedeutung haben:

$R^1$ Wasserstoff oder Halogen

$R^2$ Wasserstoff, eine $C_1$-$C_5$-Alkylgruppe, eine $C_3$-$C_4$-Alkenylgruppe, eine $C_3$-$C_4$-Alkinylgruppe, eine $C_1$-$C_5$-Halogenalkylgruppe, eine $C_3$-$C_5$-Alkoxyalkylgruppe, eine $C_3$-$C_5$-Halogenalkoxyalkylgruppe oder eine $C_5$-$C_6$-Cycloalkylgruppe

$R^3$ Wasserstoff, eine $C_1$-$C_2$-Alkylgruppe, eine Allylgruppe, eine Propargylgruppe oder eine $C_1$-$C_5$-Alkoxygruppe

$R^4$ ein Halogenatom, eine $C_1$-$C_2$-Alkylgruppe, eine $C_1$-$C_3$-Alkylthiogruppe oder eine $C_1$-$C_3$-Alkylaminogruppe,

wobei R3 nicht Wasserstoff und $C_1$-$C_2$-Alkyl bedeutet, wenn R4 $C_1$-$C_2$-Alkyl bedeutet, sowie deren umweltverträglichen Salze, sowie N-((6-Trifluormethylpyrimidin-2-yl)aminocarbonyl)-2-carboalkoxybenzolsulfonamide der allgemeinen Formel Ib,

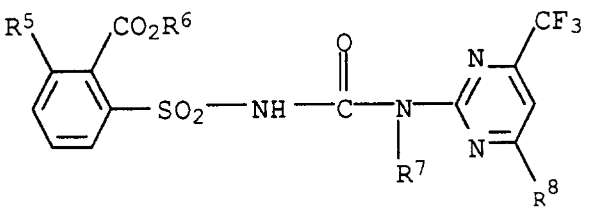

Ib

$R^5$ Wasserstoff, Fluor oder Chlor

$R^6$ eine $C_1$-$C_3$-Alkylgruppe

$R^7$ Wasserstoff oder eine Methylgruppe

$R^8$ Wasserstoff oder eine $C_1$-$C_2$-Alkoxygruppe.

Insbesondere betrifft die Erfindung N-((6-Trifluormethylpyrimidin-2-yl)aminocarbonyl)-2-carboalkoxybenzolsulfonamide der allgemeinen Formel Ia und Ib, in denen $R^2$ bzw. $R^6$ eine Methylgruppe und solche, in denen $R^4$ bzw. $R^8$ Chlor, eine Methoxygruppe, eine Methylthiogruppe bedeutet.

Des weiteren betrifft die Erfindung, Verfahren zur Herstellung der Verbindungen Ia und Ib sowie herbizide Mittel, die diese Verbindungen enthalten.

Aus US-A-4 169 719, US-A-4 563 211 und EP-A-0 084 020 sind Sulfonylharnstoffe mit herbizider Wirkung bekannt. Des weiteren beschreibt die EP-A 7687 herbizide Sulfonylharnstoffe, deren allgemeine Formel die eingangs definierten N-((6-Trifluormethylpyrimidin-2-yl)aminocarbonyl)-2-carboalkoxybenzolsulfonamide der allgemeinen Formel Ib umfaßt. Die bekannten Mittel befriedigen jedoch nicht alle Anforderungen hinsichtlich spezifischer Wirkung gegen unerwünschte Pflanzen und Verträglichkeit in Bezug auf Kulturen.

Der Erfindung lag die Aufgabe zugrunde, Sulfonylharnstoffe zu finden und zu synthetisieren, die gegenüber den bekannten Wirkstoffen dieser Herbizid-Klasse vorteilhafte Eigenschaften aufweisen.

Entsprechend dieser Aufgabe wurden die eingangs definierten N-((6-Trifluormethylpyrimidin-2-yl)-aminocarbonyl)-2-carboalkoxybenzolsulfonamide der allgemeinen Formel Ia und Ib gefunden.

Man erhält einen Sulfonylharnstoff der Formel Ia oder Ib z.B. durch Umsetzung einer entsprechenden Verbindung der Formel IIa bzw. IIb,

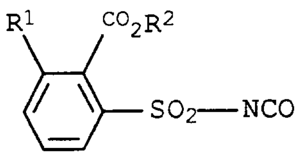

IIa

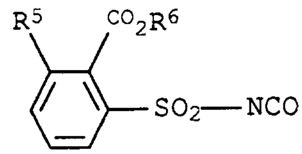

IIb

mit einer entsprechenden Verbindung der Formel IIIa bzw. IIIb,

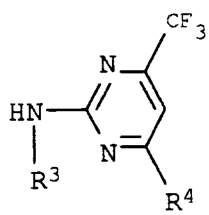

IIIa

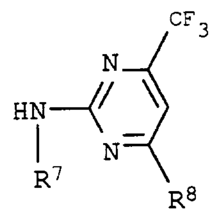

IIIb

bei einer für organische Reaktionen üblichen Temperatur von bis zu 120oC, vorzugsweise 10 bis 100oC. Die Umsetzung kann drucklos oder unter Druck kontinuierlich oder diskontinuierlich durchgeführt werden.

Die Sulfonylharnstoffe der Formel Ia und Ib können auch dadurch erhalten werden, daß man ein entsprechendes Sulfonamid der Formel IVa bzw. IVb,

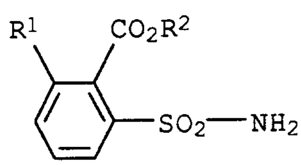

IVa

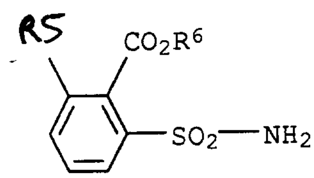

IVb

mit einem entsprechenden Phenylcarbamat der Formel Va bzw. Vb,
vorteilhaft in Gegenwart eines tertiären Amins bei einer für organische Reaktionen üblichen Temperatur von bis zu 120 ° C, vorzugsweise 10 bis 100 ° C umsetzt.

Die Sulfonylharnstoffe der Formel Ia und Ib können weiterhin dadurch erhalten werden, daß man ein entsprechendes Phenylcarbamat der Formel VIa bzw. VIb,

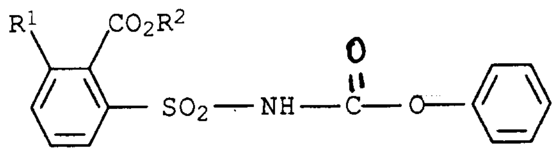

VIa

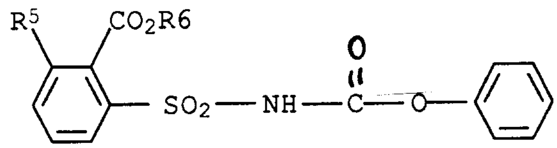

VIb

mit einem entsprechenden Amin der Formel IIIa bzw. IIIb,

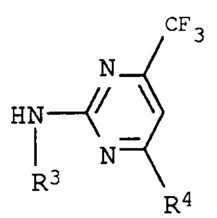

IIIa

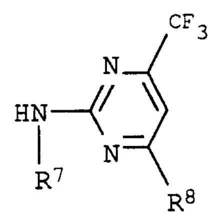

IIIb

vorteilhaft in Gegenwart eines tertiären Amins bei einer für organische Reaktionen üblichen Temperatur von bis zu 120 ° C, vorzugsweise 10 bis 100 ° C umsetzt.

Zweckmäßigerweise verwendet man für die Umsetzungen Lösungs- oder Verdünnungsmittel. Als Lösungsmittel kommen beispielsweise Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, $\beta,\beta'$-Dichlordiethylether; Nitrokohlenwasserstoffe wie o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190 ° C, Cyclohexan, Methylcyclohexan, Dekalin, Petrolether, Hexan, Ligroin, 2,2,4-Trimethylpentan, 2,2,3-Trimethylpentan, Octan; Ester z.B. Ethylacetat, Acetessigester, Isobutylacetat; Amide z.B. Formamid, Methylformamid, Dimethylformamid; Ketone, z.B. Aceton, Methylethylketon und entsprechende Gemische in Betracht. Zweckmäßig verwendet man das Lösungsmittel in einer Menge von 100 bis 2.000 Gew.%, vorzugsweise von 200 bis 700 Gew.%, bezogen auf die Ausgangsstoffe II, IV oder VI.

Die zur Umsetzung benötigten Verbindungen III oder V werden im allgemeinen in etwa stöchiometrischem Verhältnis eingesetzt. Man kann die Vorprodukte II, IV oder VI in einem Verdünnungsmittel vorlegen und dann das Vorprodukt III bzw. V zugeben.

Zweckmäßigerweise wird das Verfahren zur Herstellung der neuen Verbindungen jedoch so durchgeführt, daß man das Vorprodukt III bzw. V, vorlegt und das Vorprodukt II bzw. IV bzw. VI zugibt.

Die Umsetzungen verlaufen im Allgemeinen innerhalb von 20 Minuten bis 24 Stunden bei 0 bis 120 ° C, vorzugsweise 10 bis 100 ° C, insbesondere 20 bis 80 ° C, vollständig.

Bei den Umsetzungen, die durch die Gegenwart eines tertiären Amins als Reaktionsbeschleuniger günstig beeinflußt werden können, verwendet man z.B. Pyridin, 2,4-, 2,6-Lutidin, 2,4,6-Collidin, a,b-Picolin, p-Dimethylaminopyridin, 1,4-Diaza(2,2,2)bicyclooctan (DABCO) oder 1,8-Diazabicyclo-(5,4,0)undec-7-en in einer Menge von bis zu 1 Mol pro Mol Vorprodukt IV oder VI.

Soweit die Sulfonylharnstoffe als Säure vorliegen, kann man das Salz durch Umsetzung mit einer stöchiometrischen Menge einer wäßrigen Base oder eines Metallalkoholats, gegebenenfalls in einem organischen Lösungsmittel herstellen. Eine andere Möglichkeit ist die alkalische Verseifung entsprechender Ester.

Die Gewinnung der Zielprodukte aus den jeweiligen Reaktionsgemischen geschieht in üblicher Weise, z.B. nach Abdestillieren von Lösungsmittel oder direkt durch Absaugen. Der verbleibende Rückstand kann noch mit Wasser bzw. verdünnter Säure zur Entfernung basischer Verunreinigungen gewaschen werden. Man kann jedoch auch den Rückstand in einem mit Wasser mischbaren Lösungsmittel lösen und wie beschrieben waschen. Die gewünschten Endstoffe fallen hierbei in reiner Form an, wenn nötig können sie durch Umkristallisation oder Chromatographie gereinigt werden.

Die als Vorprodukte benötigten Verbindungen der Formel II, III, IV, V und VI lassen sich durch literaturbekannte Reaktionen darstellen. So ist die Darstellung der Ausgangstoffe III bei Gershon (H. Gershon, A.T. Grefig und A.A. Scala; J. Heterocyclic Chem. 20, 219 (1983)) beschrieben bzw. können die Ausgangsstoffe analog den dort beschriebenen Vorschriften hergestellt werden.

Im Hinblick auf die biologische Wirksamkeit sind Sulfonylharnstoffe der Formel Ia bevorzugt, in denen die Substituenten folgende Bedeutung haben:

R1 Wasserstoff oder
Halogen wie Fluor, Chlor, Brom und Jod, insbesondere Wasserstoff, Fluor, Chlor und Brom

R2 Wasserstoff,
Alkyl wie Methyl, Ethyl, Propyl, 1-Methylethyl, Butyl, 1-Metghylpropyl, 2-Methylpropyl, 1,1-Dimethylethyl, insbesondere Methyl, Ethyl, Propyl, 1-Methylethyl und 1-Methylpropyl, Alkenyl wie 1-Propenyl, 2-Propenyl, 1-Butenyl, 2-Butenyl und 3-Butenyl, insbesondere 2-Propenyl und 2-Butenyl,
Alkinyl wie 1-Propinyl, 2-Propinyl, 1-Butinyl, 2-Butinyl und 3-Butinyl, insbesondere 2-Propinyl und 2-Butinyl,
Halogenalkyl wie Fluormethyl, Difluormethyl, Trifluormethyl, Chlordifluormethyl, Dichlorfluormethyl, Trichlormethyl, 1-Fluorethyl,
2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl,
Trifluormethyl, 2,2,2-Trifluorethyl und Pentafluorethyl, Alkoxyalkyl wie 2-Methoxyethyl, 2-Methoxypropyl, 3-Methoxypropyl,
2-Methoxy-1-methylethyl, Ethoxymethyl, 2-Ethoxyethyl, 2-Ethoxypropyl,
3-Ethoxypropyl, 2-Ethoxy-1-methylethyl und 1-Ethoxy-1-methylethyl,
insbesondere Methoxyethyl und Ethoxyethyl, Halogenalkoxyalkyl wie Difluormethoxyethyl, Trifluormethoxyethyl,
Chlordifluormethoxyethyl, Dichlorfluormethoxyethyl, 1-Fluorethoxyethyl, 2-Fluorethoxyethyl, 2,2-Difluorethoxyethyl,
1,1,2,2-Tetrafluorethoxyethyl, 2,2,2-Trifluorethoxyethyl, 2-Chlor-1,1,2-trifluorethoxyethyl und Pentafluorethoxyethyl, insbesondere Trifluormethoxyethyl oder
Cycloalkyl wie Cyclopentyl und Cyclohexyl

R3 Wasserstoff, Methyl, Ethyl, Allyl, Propargyl oder Alkoxy wie Methoxy,
Ethoxy, Propoxy, 1-Methylethoxy, Butoxy, 1-Methylpropoxy, 2-Methylpropoxy und 1,1-Dimethylethoxy, insbesondere Wasserstoff,
Methyl, Alkyl, Propargyl, Methoxy und Ethoxy

R4 Halogen wie bei R1 genannt, insbesondere Fluor, Chlor und Brom,
Alkylthio wie Methylthio Ethylthio, Propylthio und 1-Methylethylthio,
insbesondere Methylthio, Ethylthio und 1-Methylethylthio oder Alkylamino wie Methylamino, Ethylamino, Propylamino und 1-Methylethylamino, insbesondere Methylamino und Ethylamino.

Von den Sulfonylharnstoffen der Formel Ib sind im Hinblick auf die biologische Wirksamkeit insbesondere solche bevorzugt, in denen die Substituenten folgende Bedeutung haben:

$R^5$ Wasserstoff, Fluor oder Chlor
$R^6$ insbesondere Methyl oder Ethyl
$R^7$ Wasserstoff oder Methyl und
$R^8$ Wasserstoff, Methoxy oder Ethoxy.

Im Hinblick auf die biologische Wirksamkeit seien spezielle Beispiele für herbizide Sulfonylharnstoffe der allgemeinen Formeln Ia und Ib in den folgenden Tabellen a) und b) zusammengestellt.

Tabelle a) - Sulfonylharnstoffe der Struktur Ia

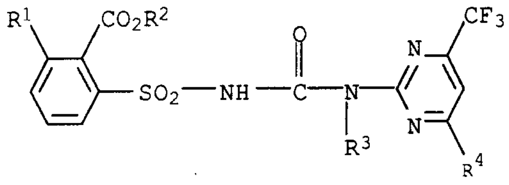

Ia

| $R^1$ | $R^2$ | $R^3$ | $R^4$ |
|---|---|---|---|
| H | $CH_3$ | H | Cl |
| H | $C_2H_5$ | H | Cl |
| H | $n-C_3H_7$ | H | Cl |
| H | $n-C_4H_9$ | H | Cl |
| H | $i-C_3H_7$ | H | Cl |
| H | $sec-C_4H_9$ | H | Cl |
| H | $CH_2CH=CH_2$ | H | Cl |
| H | $CH_2CH{\equiv}CH$ | H | Cl |
| H | $CH_2CH_2Cl$ | H | Cl |
| H | $CH_2CH_2OCH_3$ | H | Cl |
| H | Cyclohexyl | H | Cl |
| H | $CH_3$ | H | $NHCH_3$ |
| H | $C_2H_5$ | H | $NHCH_3$ |
| H | $n-C_3H_7$ | H | $NHCH_3$ |
| H | $n-C_4H_9$ | H | $NHCH_3$ |
| H | $i-C_3H_7$ | H | $NHCH_3$ |
| H | $sec-C_4H_9$ | H | $NHCH_3$ |
| H | $CH_2CH=CH_2$ | H | $NHCH_3$ |
| H | $CH_2CH{\equiv}CH$ | H | $NHCH_3$ |
| H | $CH_2CH_2Cl$ | H | $NHCH_3$ |
| H | $CH_2CH_2OCH_3$ | H | $NHCH_3$ |
| H | Cyclohexyl | H | $NHCH_3$ |
| H | $CH_3$ | H | $SCH_3$ |
| H | $C_2H_5$ | H | $SCH_3$ |
| H | $n-C_3H_7$ | H | $SCH_3$ |
| H | $n-C_4H_9$ | H | $SCH_3$ |
| H | $i-C_3H_7$ | H | $SCH_3$ |

| $R^1$ | $R^2$ | $R^3$ | $R^4$ |
|---|---|---|---|
| H | sec-$C_4H_9$ | H | $SCH_3$ |
| H | $CH_2CH{=}CH_2$ | H | $SCH_3$ |
| H | $CH_2CH{\equiv}CH$ | H | $SCH_3$ |
| H | $CH_2CH_2Cl$ | H | $SCH_3$ |
| H | $CH_2CH_2OCH_3$ | H | $SCH_3$ |
| H | Cyclohexyl | H | $SCH_3$ |
| H | $CH_3$ | H | $SC_2H_5$ |
| H | $C_2H_5$ | H | $SC_2H_5$ |
| H | n-$C_3H_7$ | H | $SC_2H_5$ |
| H | n-$C_4H_9$ | H | $SC_2H_5$ |
| H | i-$C_3H_7$ | H | $SC_2H_5$ |
| H | sec-$C_4H_9$ | H | $SC_2H_5$ |
| H | $CH_2CH{=}CH_2$ | H | $SC_2H_5$ |
| H | $CH_2CH{\equiv}CH$ | H | $SC_2H_5$ |
| H | $CH_2CH_2Cl$ | H | $SC_2H_5$ |
| H | $CH_2CH_2OCH_3$ | H | $SC_2H_5$ |
| H | Cyclohexyl | H | $SC_2H_5$ |
| H | n-$C_3H_7$ | H | $SC_3H_7$ |
| H | $CH_3$ | $CH_3$ | Cl |
| H | $C_2H_5$ | $CH_3$ | Cl |
| H | n-$C_3H_7$ | $CH_3$ | Cl |
| H | n-$C_4H_9$ | $CH_3$ | Cl |
| H | i-$C_3H_7$ | $CH_3$ | Cl |
| H | sec-$C_4H_9$ | $CH_3$ | Cl |
| H | $CH_2CH{=}CH_2$ | $CH_3$ | Cl |
| H | $CH_2CH{\equiv}CH$ | $CH_3$ | Cl |
| H | $CH_2CH_2Cl$ | $CH_3$ | Cl |
| H | $CH_2CH_2OCH_3$ | $CH_3$ | Cl |
| H | Cyclohexyl | $CH_3$ | Cl |
| H | $CH_3$ | $CH_3$ | $NHCH_3$ |
| H | $C_2H_5$ | $CH_3$ | $NHCH_3$ |
| H | n-$C_3H_7$ | $CH_3$ | $NHCH_3$ |
| H | n-$C_4H_9$ | $CH_3$ | $NHCH_3$ |
| H | i-$C_3H_7$ | $CH_3$ | $NHCH_3$ |

| $R^1$ | $R^2$ | $R^3$ | $R^4$ |
|---|---|---|---|
| H | sec-$C_4H_9$ | $CH_3$ | $NHCH_3$ |
| H | $CH_2CH{=}CH_2$ | $CH_3$ | $NHCH_3$ |
| H | $CH_2CH{\equiv}CH$ | $CH_3$ | $NHCH_3$ |
| H | $CH_2CH_2Cl$ | $CH_3$ | $NHCH_3$ |
| H | $CH_2CH_2OCH_3$ | $CH_3$ | $NHCH_3$ |
| H | Cyclohexyl | $CH_3$ | $NHCH_3$ |
| H | $CH_3$ | $OCH_3$ | Cl |
| H | $C_2H_5$ | $OCH_3$ | Cl |
| H | n-$C_3H_7$ | $OCH_3$ | Cl |
| H | n-$C_4H_9$ | $OCH_3$ | Cl |
| H | i-$C_3H_7$ | $OCH_3$ | Cl |
| H | sec-$C_4H_9$ | $OCH_3$ | Cl |
| H | $CH_2CH{=}CH_2$ | $OCH_3$ | Cl |
| H | $CH_2CH{\equiv}CH$ | $OCH_3$ | Cl |
| H | $CH_2CH_2Cl$ | $OCH_3$ | Cl |
| H | $CH_2CH_2OCH_3$ | $OCH_3$ | Cl |
| H | Cyclohexyl | $OCH_3$ | Cl |
| H | $CH_3$ | $OCH_3$ | $CH_3$ |
| H | $C_2H_5$ | $OCH_3$ | $CH_3$ |
| H | n-$C_3H_7$ | $OCH_3$ | $CH_3$ |
| H | n-$C_4H_9$ | $OCH_3$ | $CH_3$ |
| H | i-$C_3H_7$ | $OCH_3$ | $CH_3$ |
| H | sec-$C_4H_9$ | $OCH_3$ | $CH_3$ |
| H | $CH_2CH{=}CH_2$ | $OCH_3$ | $CH_3$ |
| H | $CH_2C{\equiv}CH$ | $OCH_3$ | $CH_3$ |
| H | $CH_2CH_2Cl$ | $OCH_3$ | $CH_3$ |
| H | $CH_2CH_2OCH_3$ | $OCH_3$ | $CH_3$ |
| H | Cyclohexyl | $OCH_3$ | $CH_3$ |
| H | $CH_3$ | $OCH_3$ | $NHCH_3$ |
| H | $C_2H_5$ | $OCH_3$ | $NHCH_3$ |
| H | n-$C_3H_7$ | $OCH_3$ | $NHCH_3$ |
| H | n-$C_4H_9$ | $OCH_3$ | $NHCH_3$ |
| H | i-$C_3H_7$ | $OCH_3$ | $NHCH_3$ |
| H | sec-$C_4H_9$ | $OCH_3$ | $NHCH_3$ |

| $R^1$ | $R^2$ | $R^3$ | $R^4$ |
|---|---|---|---|
| H | $CH_2CH{=}CH_2$ | $OCH_3$ | $NHCH_3$ |
| H | $CH_2C{\equiv}CH$ | $OCH_3$ | $NHCH_3$ |
| H | $CH_2CH_2Cl$ | $OCH_3$ | $NHCH_3$ |
| H | $CH_2CH_2OCH_3$ | $OCH_3$ | $NHCH_3$ |
| H | Cyclohexyl | $OCH_3$ | $NHCH_3$ |
| H | $CH_3$ | $CH_2CH{=}CH_2$ | Cl |
| H | $C_2H_5$ | $CH_2CH{=}CH_2$ | Cl |
| H | $n-C_3H_7$ | $CH_2CH{=}CH_2$ | Cl |
| H | $n-C_4H_9$ | $CH_2CH{=}CH_2$ | Cl |
| H | $i-C_3H_7$ | $CH_2CH{=}CH_2$ | Cl |
| H | $sec-C_4H_9$ | $CH_2CH{=}CH_2$ | Cl |
| H | $CH_2CH{=}CH_2$ | $CH_2CH{=}CH_2$ | Cl |
| H | $CH_2C{\equiv}CH$ | $CH_2CH{=}CH_2$ | Cl |
| H | $CH_2CH_2Cl$ | $CH_2CH{=}CH_2$ | Cl |
| H | $CH_2CH_2OCH_3$ | $CH_2CH{=}CH_2$ | Cl |
| H | Cyclohexyl | $CH_2CH{=}CH_2$ | Cl |
| H | $CH_3$ | $CH_2CH{=}CH_2$ | $CH_3$ |
| H | $C_2H_5$ | $CH_2CH{=}CH_2$ | $CH_3$ |
| H | $n-C_3H_7$ | $CH_2CH{=}CH_2$ | $CH_3$ |
| H | $n-C_4H_9$ | $CH_2CH{=}CH_2$ | $CH_3$ |
| H | $i-C_3H_7$ | $OCH_3$ | $CH_3$ |
| H | $sec-C_4H_9$ | $OCH_3$ | $CH_3$ |
| H | $CH_2CH{=}CH_2$ | $OCH_3$ | $CH_3$ |
| H | $CH_2C{\equiv}CH$ | $OCH_3$ | $CH_3$ |
| H | $CH_2CH_2Cl$ | $OCH_3$ | $CH_3$ |
| H | $CH_2CH_2OCH_3$ | $OCH_3$ | $CH_3$ |
| H | Cyclohexyl | $OCH_3$ | $CH_3$ |
| H | $CH_3$ | $CH_2CH{=}CH_2$ | $NHCH_3$ |
| H | $C_2H_5$ | $CH_2CH{=}CH_2$ | $NHCH_3$ |
| H | $n-C_3H_7$ | $CH_2CH{=}CH_2$ | $NHCH_3$ |
| H | $n-C_4H_9$ | $CH_2CH{=}CH_2$ | $NHCH_3$ |
| H | $i-C_3H_7$ | $CH_2CH{=}CH_2$ | $NHCH_3$ |
| H | $sec-C_4H_9$ | $CH_2CH{=}CH_2$ | $NHCH_3$ |
| H | $CH_2CH{=}CH_2$ | $CH_2CH{=}CH_2$ | $NHCH_3$ |

| $R^1$ | $R^2$ | $R^3$ | $R^4$ |
|---|---|---|---|
| H | $CH_2C\equiv CH$ | $CH_2CH=CH_2$ | $NHCH_3$ |
| H | $CH_2CH_2Cl$ | $CH_2CH=CH_2$ | $NHCH_3$ |
| H | $CH_2CH_2OCH_3$ | $CH_2CH=CH_2$ | $NHCH_3$ |
| H | Cyclohexyl | $CH_2CH=CH_2$ | $NHCH_3$ |
| Cl | $CH_3$ | H | Cl |
| Cl | $C_2H_5$ | H | Cl |
| Cl | $n-C_3H_7$ | H | Cl |
| Cl | $n-C_4H_9$ | H | Cl |
| Cl | $i-C_3H_7$ | H | Cl |
| Cl | $sec-C_4H_9$ | H | Cl |
| Cl | $CH_2CH=CH_2$ | H | Cl |
| Cl | $CH_2C\equiv CH$ | H | Cl |
| Cl | $CH_2CH_2Cl$ | H | Cl |
| Cl | $CH_2CH_2OCH_3$ | H | Cl |
| Cl | Cyclohexyl | H | Cl |
| Cl | $CH_3$ | $CH_3$ | Cl |
| Cl | $C_2H_5$ | $CH_3$ | Cl |
| Cl | $n-C_3H_7$ | $CH_3$ | Cl |
| Cl | $n-C_4H_9$ | $CH_3$ | Cl |
| Cl | $i-C_3H_7$ | $CH_3$ | Cl |
| Cl | $sec-C_4H_9$ | $CH_3$ | Cl |
| Cl | $CH_2CH=CH_2$ | $CH_3$ | Cl |
| Cl | $CH_2C\equiv CH$ | $CH_3$ | Cl |
| Cl | $CH_2CH_2Cl$ | $CH_3$ | Cl |
| Cl | $CH_2CH_2OCH_3$ | $CH_3$ | Cl |
| Cl | Cyclohexyl | $CH_3$ | Cl |
| F | $CH_3$ | H | Cl |
| F | $C_2H_5$ | H | Cl |
| F | $n-C_3H_7$ | H | Cl |
| F | $n-C_4H_9$ | H | Cl |
| F | $i-C_3H_7$ | H | Cl |
| F | $sec-C_4H_9$ | H | Cl |
| F | $CH_2CH=CH_2$ | H | Cl |
| F | $CH_2C\equiv CH$ | H | Cl |

| $R^1$ | $R^2$ | $R^3$ | $R^4$ |
|---|---|---|---|
| F | $CH_2CH_2Cl$ | H | Cl |
| F | $CH_2CH_2OCH_3$ | H | Cl |
| F | Cyclohexyl | H | Cl |
| F | $CH_3$ | H | $SCH_3$ |
| F | $CH_3$ | $CH_3$ | Cl |
| F | $C_2H_5$ | $CH_3$ | Cl |
| F | $n-C_3H_7$ | $CH_3$ | Cl |
| F | $n-C_4H_9$ | $CH_3$ | Cl |
| F | $i-C_3H_7$ | $CH_3$ | Cl |
| F | $sec-C_4H_9$ | $CH_3$ | Cl |
| F | $CH_2CH=CH_2$ | $CH_3$ | Cl |
| F | $CH_2C\equiv CH$ | $CH_3$ | Cl |
| F | $CH_2CH_2Cl$ | $CH_3$ | Cl |
| F | $CH_2CH_2OCH_3$ | $CH_3$ | Cl |
| F | Cyclohexyl | $CH_3$ | Cl |

Tabelle b) - Sulfonylharnstoffe der Struktur Ib

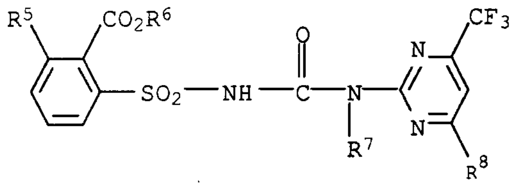

Ib

| $R^5$ | $R^6$ | $R^7$ | $R^8$ |
|---|---|---|---|
| H | $CH_3$ | H | $OCH_3$ |
| H | $C_2H_5$ | H | $OCH_3$ |
| H | n-$C_3H_7$ | H | $OCH_3$ |
| H | i-$C_3H_7$ | H | $OCH_3$ |
| H | $CH_3$ | H | $OC_2H_5$ |
| H | $C_2H_5$ | H | $OC_2H_5$ |
| H | n-$C_3H_7$ | H | $OC_2H_5$ |
| H | i-$C_3H_7$ | H | $OC_2H_5$ |
| H | $CH_3$ | $CH_3$ | $OCH_3$ |
| H | $C_2H_5$ | $CH_3$ | $OCH_3$ |
| H | n-$C_3H_7$ | $CH_3$ | $OCH_3$ |
| H | i-$C_3H_7$ | $CH_3$ | $OCH_3$ |
| Cl | $CH_3$ | H | $OCH_3$ |
| Cl | $C_2H_5$ | H | $OCH_3$ |
| Cl | n-$C_3H_7$ | H | $OCH_3$ |
| Cl | i-$C_3H_7$ | H | $OCH_3$ |
| Cl | $CH_3$ | $CH_3$ | $OCH_3$ |
| Cl | $C_2H_5$ | $CH_3$ | $OCH_3$ |
| Cl | n-$C_3H_7$ | $CH_3$ | $OCH_3$ |
| Cl | i-$C_3H_7$ | $CH_3$ | $OCH_3$ |
| F | $CH_3$ | H | $OCH_3$ |
| F | $C_2H_5$ | H | $OCH_3$ |
| F | n-$C_3H_7$ | H | $OCH_3$ |
| F | i-$C_3H_7$ | H | $OCH_3$ |
| F | $CH_3$ | $CH_3$ | $OCH_3$ |
| F | $C_2H_5$ | $CH_3$ | $OCH_3$ |
| F | n-$C_3H_7$ | $CH_3$ | $OCH_3$ |
| F | i-$C_3H_7$ | $CH_3$ | $OCH_3$ |

Die N-((6-Trifluormethylpyrimidin-2-yl)aminocarbonyl)-2-carboalkoxybenzol-sulfonamide der allgemeinen Formel Ia und Ib, bzw. die sie enthaltenden herbiziden Mittel sowie deren umweltverträglichen Salze von Alkalimetallen und Erdalkalimetallen können Schadpflanzen sehr gut bekämpfen, ohne die Kulturpflanzen zu schädigen, ein Effekt, der vor allem auch bei niedrigen Aufwandmengen auftritt. Sie können beispielsweise in Form von direkt versprühbaren Lösungen, Pulvern, Suspensionen, auch hochprozentigen wäßrigen,

öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen Ia und Ib eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkyl-arylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff.

Die Wirkstoffe können beispielsweise wie folgt formuliert werden:

I. Man vermischt 90 Gewichtsteile der Verbindung Nr. 1.012 mit 10 Gewichtsteilen N-Methyl-a-pyrrolidon und erhält eine Lösung, die zur Anwendung in Form kleinster Tropfen geeignet ist.

II. 20 Gewichtsteile der Verbindung Nr. 1.005 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.017 werden in einer Mischung gelöst, die aus 40 Gewichtsteilen Cyclohexanon, 30 Gewichtsteilen Isobutanol, 20 Gewichtsteilen des Anlagerungs-Produktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.012 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 1.010 werden mit 3 Gewichtsteilen des Natriumsalzes der Diisobutylnaphthalin-a-sulfonsäure, 17 Gewichtsteilen des Natriumsalzes einer Ligninsulfonsäure aus

einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wassererhält man eine Spritzbrühe, die 0,1 Gew.% des Wirkstoffs enthält.

VI. 3 Gewichtsteile des Wirkstoffs Nr. 1.009 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 2.001 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 2.009 werden mit 2 Gewichtsteilen Calciumsalz der Dodecylbenzolsulfonsäure, 8 Gewichtsteilen Fettalkohol-polyglykolether, 2 Gewichtsteilen Natriumsalz einesPhenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0 kg/ha, vorzugsweise 0,005 bis 1,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kühlrübe |
| Brassica rapa var. silvestris | Rüben |
| Camellia sinensis | Teestrauch |
| Carthamus tinctorius | Saflor-Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis Guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |

| Botanischer Name | Deutscher Name |
|---|---|
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicaco sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (S. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die Cyclohexenonoximether I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäure sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Synthesebeispiel

2-(((4-methoxy-6-trifluormethyl-1,3-pyrimidin-2-yl)-aminocarbonyl)-aminosulfonyl)-benzoesäuremethylester

6,5 g 2-(Isocyanatosulfonyl)benzoesäuremethylester in 50 ml abs. Acetonitril wurden bei Raumtemperatur innerhalb 10 Minuten unter Rühren und Stickstoff zu einer Suspension von 5,2 g 2-Amino-4-methoxy-6-trifluor-methylpyrimidin in 100 ml abs. Acetonitril gegeben, wobei eine Temperaturerhöhung von 10 °C eintrat. Nach 13 Stunden Rühren bei 70 °C wurde die Reaktionsmischung eingeengt und der Rückstand in Dichlormethan aufgenommen. Die organische Phase wurde einmal mit 1n Salzsäure und einmal mit 1 N Natronlauge gewaschen, mit Natriumsulfat getrocknet und filtriert. Nach Abziehen des Solvens erhielt man 9,7 g der Titelverbindung mit dem Schmelzpunkt von 150 - 151 °C.

Durch entsprechende Abwandlung des vorstehenden Beispiels wurden die in den folgenden Tabellen aufgeführten Verbindungen hergestellt.

Wirkstofftabelle 1

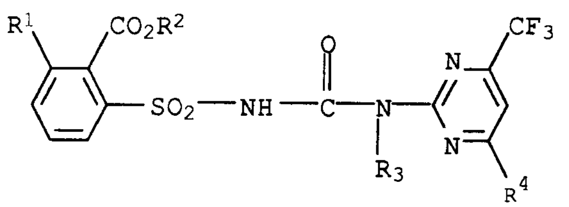

Ia

| Wirkstoff Nr. | $R^1$ | $R^2$ | $R^3$ | $R^4$ | Fp(°) |
|---|---|---|---|---|---|
| 1.001 | H | $CH_3$ | H | Cl | 176–177 |
| 1.003 | H | $CH_3$ | H | $NHCH_3$ | 115–117 |
| 1.004 | H | $CH_3$ | H | $SCH_3$ | 155–157 |
| 1.005 | H | $CH_3$ | H | $SCH_2CH_3$ | 71–73 |
| 1.006 | H | $CH_2CH_3$ | H | $SCH_3$ | 114–117 |
| 1.007 | H | $(CH_3)_2CH_3$ | H | $SCH_3$ | 146–148 |
| 1.008 | H | $(CH_3)_2CH_3$ | H | $S(CH_2)_2CH_3$ | 146–148 |
| 1.009 | H | $CH_3$ | H | Cl | 86–87 |
| 1.010 | H | $CH_3$ | H | $SCH_3$ | 181–187 |
| 1.011 | H | $CH_3$ | H | $S(CH_2)_2CH_3$ | 188–189 |
| 1.012 | H | $CH_3$ | H | $SCH_3$ | 204–206 |

Wirkstofftabelle 2

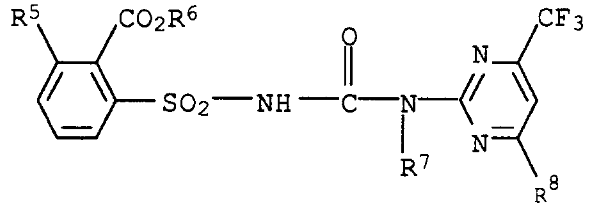

Ib

| Wirkstoff Nr. | $R^5$ | $R^6$ | $R^7$ | $R^8$ | Fp (°) |
|---|---|---|---|---|---|
| 2.001 | H | $CH_3$ | H | H | 160–168 |
| 2.002 | H | $CH_3$ | H | $OCH_3$ | 150–151 |
| 2.003 | H | $CH_3$ | H | $OCH_2CH_3$ | 150–152 |
| 2.004 | H | $CH_2CH_3$ | H | $OCH_3$ | 147–149 |
| 2.005 | H | $CH_2CH_3$ | H | $OCH_2CH_3$ | 130–132 |
| 2.006 | H | $(CH_2)_2CH_3$ | H | $OCH_3$ | 200–202 |
| 2.007 | H | $CH_3$ | $CH_3$ | $OCH_3$ | 137–139 |
| 2.008 | Cl | $CH_3$ | H | $OCH_3$ | 208–211 |
| 2.009 | F | $CH_3$ | H | $OCH_3$ | 179–182 |

Die herbizide Wirkung der N-[(6-trifluormethylpyrimidin-2-yl)-amino-carbonyl]-2-carboalkoxybenzolsulfonamide der Formel Ia und Ib auf das Wachstum der Testpflanzen wird durch folgende Gewächshausversuche gezeigt.

Zur Anzucht der Testpflanzen dienen Plastikblumentöpfe mit 300 cm3 Inhalt und lehmigem Sand mit etwa 3,0 % Humus als Substrat. Die Samen der Testpflanzen werden nach Arten getrennt flach eingesät.

Bei Vorauflaufbehandlung werden die aufbereiteten Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird. Die Aufwandmengen betragen 0,03 kg/ha.

Zum Zwecke der Nachauflaufbehandlung zieht man die Testpflanzen je nach Wuchsform erst bis zu einer Wuchshöhe von 3 bis 15 cm an und behandelt sie danach. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Eine Abdeckung unterbleibt bei der Nachauflaufbehandlung. Die Aufwandmengen für die Nachauflaufbehandlung betragen 0,03 und 0,06 kg/ha aktive Substanz (a.S).

Die Versuchsgefäße werden im Gewächshaus aufgestellt, wobei für wärmeliebende Arten wärmere Bereiche (20 bis 35 °C) und für solche gemäßigter Klimate 10 bis 20 °C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Abkürz. | Lateinischer Name | Deutscher Name | Englischer Name |
|---|---|---|---|
| ABUTH | Abutilon theophrasti | chinesischer Hanf | velvet leaf |
| AMARE | Amaranthus retroflexus | zurückgekrümmter Fuchsschwanz | redroot pigweed |
| BROIN | Bromus inermis | unbegrannte Trespe | smooth broome |
| CHEAL | Chenopodium album | weißer Gänsefuß | lambsquarters (goosefoot) |
| CYPIR | Cyperus iria | | flatsedge, rice |
| ECHCG | Echinochlora crus-galli | Hühnerhirse | barnyardgrass |
| GALAP | Galium aparine | Klettenlabkraut | catchweed bedstraw |
| LAMAM | Lamium amplexicaule | stengelumfassende Taubnessel | henbit |
| POAAN | Poa annua | einjährige Rispe | annual bluegrass |
| STEME | Stellaria media | Vogelsternmiere | chickweed |
| VERSS | Veronica spp. | Ehrenpreisarten | speedwell |

Als Vergleichsmittel werden Wirkstoffe aus der US-A 4 169 719 der nachstehender Struktur A bzw. B verwendet und den neuen Wirkstoffen, bei denen $R^1$ eine Carbalkoxystruktur hat, gegenübergestellt.

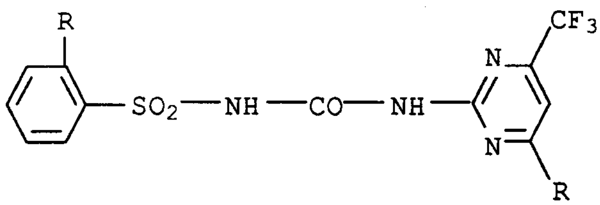

A, B

| Vergleichsmittel | $R^1$ | $R^2$ | Bsp. in US-A 4 169 719 |
|---|---|---|---|
| A | Cl | $OCH_3$ | Tab. I - E |
| B | Cl | $CH_3$ | Tab. I - D |

Mit 0,03 bzw. 0,06 kg/ha a.S. (aktive Substanz) im Nachauflaufverfahren eingesetzt, lassen sich mit Bsp. 2.002 bzw. Bsp. 1.004 breitblättrige unerwünschte Pflanzen sehr gut bekämpfen, während A und B bei diesen Konzentrationen praktisch wirkungslos sind.

Mit Bsp. 2.002 lassen sich im Vorauflaufverfahren, eingesetzt mit 0,03 kg/ha a.S., dikotyle und monokotyle unerwünschte Pflanzen sehr gut bekämpfen; auch hier sind A und B praktisch unwirksam.

Tabelle A

Beispiele zur Bekämpfung unerwünschter breitblättriger Pflanzen bei Nachauflaufapplikation von 0,03 kg/ha a.S. im Gewächshaus

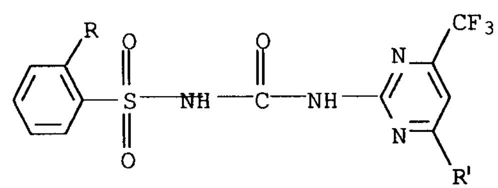

I

| Wirkstoff Nr. | R | R′ (= $R^8$) | Testpflanzen und Schädigung (%) CYPIR | ECHCG | ABUTH | AMARE | GALAP | VERSS |
|---|---|---|---|---|---|---|---|---|
| 2.002 | $CO_2CH_3$ | $OCH_3$ | 100 | 98 | 98 | 100 | 100 | 100 |
| A* | Cl | $OCH_3$ | 0 | 0 | 0 | 0 | 0 | 0 |
| B** | Cl | $CH_3$ | 0 | 0 | 0 | 40 | 50 | 0 |

A* = Aus US-A 4 169 719 (Tab. I-E)
B** = Aus US-A 4 169 719 (Tab. I-D)

Tabelle B

Beispiele zur Bekämpfung unerwünschter breitblättriger Pflanzen bei Vorauflaufapplikation von 0,03 kg/ha a.S. im Gewächshaus

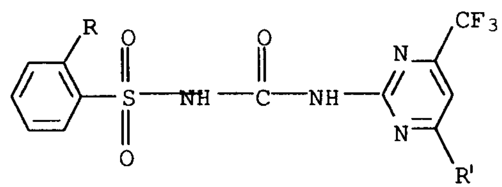

I,

| Wirkstoff Nr. | R | R' (= $R^8$) | Testpflanzen und Schädigung (%) BROIN | ECHCG | POAAN | AMARE | CHEAL | GALAP | VERSS | CYPIR |
|---|---|---|---|---|---|---|---|---|---|---|
| 2.002 | $CO_2CH_3$ | $OCH_3$ | 98 | 98 | 98 | 98 | 98 | 95 | 100 | 100 |
| A* | Cl | $OCH_3$ | 0 | 0 | 0 | 0 | 0 | 0 | 0 | 0 |
| B** | Cl | $CH_3$ | 0 | 0 | 0 | 0 | 0 | 0 | 0 | 0 |

A* = Aus US-A 4 169 719 (Tab. I-E)
B** = Aus US-A 4 169 719 (Tab. I-D)

Tabelle C

Beispiel zur Bekämpfung von unerwünschten breitblättrigen Pflanzen bei Nachauflaufapplikation von 0,06 kg/ha des Wirkstoffs Nr. 1.004 im Gewächshaus

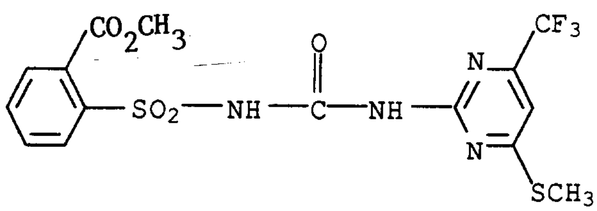

| Testpflanzen | Schädigung in % |
|---|---|
| ABUTH | 100 |
| AMARE | 100 |
| CHEAL | 100 |
| LAMAM | 100 |
| STEME | 100 |

**Patentansprüche**

1. N-((6-Trifluormethylpyrimidin-2-yl)aminocarbonyl)-2-carboalkoxybenzol-sulfonamide der allgemeinen Formel Ia,

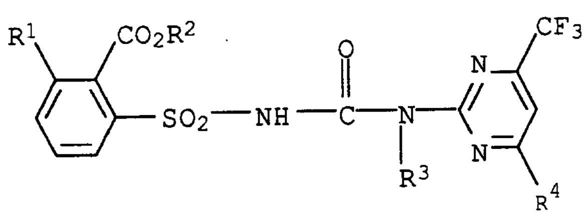

Ia

in der die Substituenten folgende Bedeutung haben:

$R^1$ Wasserstoff oder Halogen

$R^2$ Wasserstoff, eine $C_1$-$C_5$-Alkylgruppe, eine $C_3$-$C_4$-Alkenylgruppe, eine $C_3$-$C_4$-Alkinylgruppe, eine $C_1$-$C_5$-Halogenalkylgruppe, eine $C_3$-$C_5$-Alkoxyalkylgruppe, eine $C_3$-$C_5$-Halogenalkoxyalkylgruppe oder eine $C_5$-$C_6$-Cycloalkylgruppe

$R^3$ Wasserstoff, eine $C_1$-$C_2$-Alkylgruppe, eine Allylgruppe, eine Propargylgruppe oder eine $C_1$-$C_5$-Alkoxygruppe

$R^4$ ein Halogenatom, eine $C_1$-$C_2$-Alkylgruppe, eine $C_1$-$C_3$-Alkylthiogruppe oder eine $C_1$-$C_3$-Alkylaminogruppe,

wobei R3 nicht Wasserstoff und $C_1$-$C_2$-Alkyl bedeutet, wenn R4 $C_1$-$C_2$-Alkyl bedeutet, sowie deren umweltverträglichen Salze.

2. N-((6-Trifluormethylpyrimidin-2-yl)aminocarbonyl)-2-carboalkoxybenzolsulfonamide der allgemeinen Formel Ib,

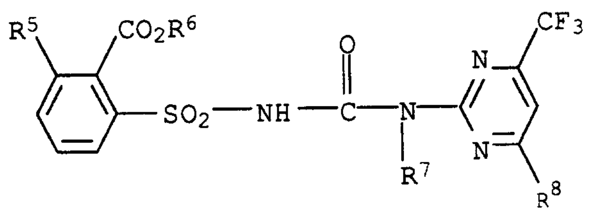

Ib

in der die Substituenten folgende Bedeutung haben:

- $R^5$ Wasserstoff, Fluor oder Chlor
- $R^6$ eine $C_1$-$C_3$-Alkylgruppe
- $R^7$ Wasserstoff oder eine Methylgruppe
- $R^8$ Wasserstoff oder eine $C_1$-$C_2$-Alkoxygruppe.

3. N-((6-Trifluormethylpyrimidin-2-yl)aminocarbonyl)-2-carboalkoxybenzolsulfonamide der Formel Ia und Ib gemäß Anspruch 1 und 2, in denen $R^2$ bzw. $R^6$ eine Methylgruppe bedeutet.,

4. Verfahren zur Herstellung von N-((6-Trifluormethylpyrimidin-2-yl)aminocarbonyl)-2-carboalkoxybenzolsulfonamiden der Formeln Ia und Ib gemäß Anspruch 1 und 2, dadurch gekennzeichnet, daß man einen entsprechenden 2-(Isocyanatosulfonyl)benzoesäureester der Formel IIa bzw. IIb,

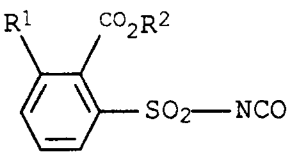

IIa

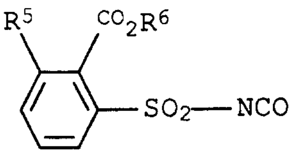

IIb

mit einem Amin der Formel IIIa bzw. IIIb,

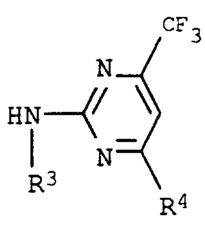

IIIa

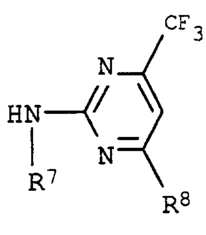

IIIb

in an sich bekannter Weise umsetzt.

5. Verfahren zur Herstellung von N-((6-Trifluormethylpyrimidin-2-yl)aminocarbonyl)-2-carboalkoxybenzolsulfonamiden der Formeln Ia und Ib gemäß Anspruch 1 und 2, dadurch gekennzeichnet, daß man ein entsprechendes Sulfonamid der Formel IVa bzw. IVb,

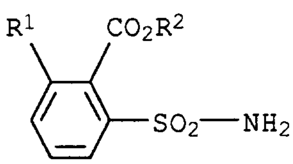

IVa

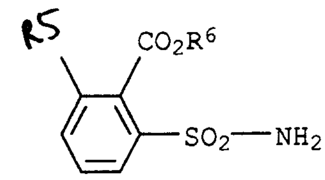

IVb

mit einem entsprechenden Phenylcarbamat der Formel Va bzw. Vb,

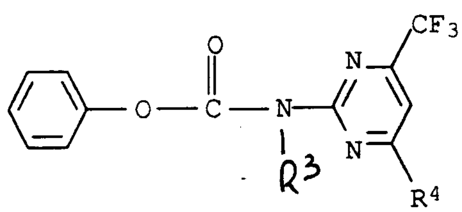

Va

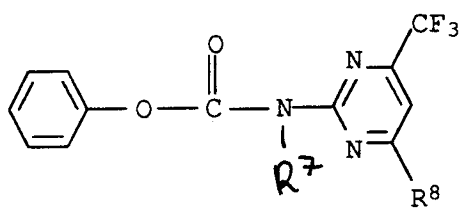

Vb

in an sich bekannter Weise umsetzt.

**6.** Verfahren zur Herstellung von N-((6-Trifluormethylpyrimidin-2-yl)aminocarbonyl)-2-carboalkoxybenzolsulfonamiden der Formeln Ia und Ib gemäß Anspruch 1 und 2, dadurch gekennzeichnet, daß man ein entsprechendes Phenylcarbamat der Formel VIA bzw. VIb,

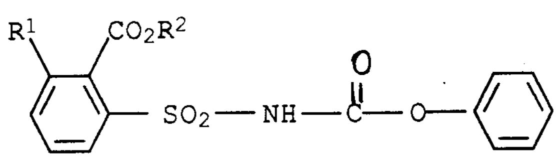

VIa

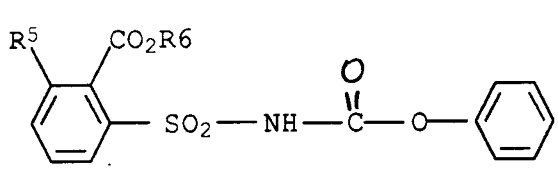

VIb

in an sich bekannter Weise mit einem entsprechenden Amin der Formel IIIa bzw. IIIb gemäß Anspruch 4 umsetzt.

**7.** Herbizides Mittel, enthaltend ein N-((6-Trifluormethylpyrimidin-2-yl)-aminocarbonyl)-2-carboalkoxybenzolsulfonamid der Formeln Ia und Ib gemäß den Ansprüchen 1 und 2 oder dessen landwirtschaftlich brauchbare Salze neben hierfür üblichen Hilfs- und Verdünnungsmitteln.

**8.** Verfahren zur Bekämpfung unerwünschten Pflanzenwuchses, dadurch ge-kennzeichnet, daß man ein N-((6-Trifluormethylpyrimidin-2-yl)-amino-carbonyl)-2-carboalkoxybenzol-sulfonamid der Formeln Ia und Ib

gemäß den Ansprüchen 1 und 2 oder dessen landwirtschaftlich brauchbare Salze auf die Pflanzen und/oder ihren Lebensraum einwirken läßt.

**9.** Mittel zur Beeinflussung des Pflanzenwuchses, enthaltend neben üblichen Hilfs- und Verdünnungsmitteln einen Sulfonylharnstoff der Formel Ia und Ib gemäß den Ansprüchen 1 und 2 oder dessen landwirtschaftlich brauchbare Salze.

**10.** Verfahren zur Beeinflussung des Pflanzenwuchses, dadurch gekennzeichnet, daß man ein N-((6-Trifluormethylpyrimidin-2-yl)-aminocarbonyl)-2-carboalkoxybenzol-sulfonamid der Formeln Ia und Ib gemäß den Ansprüchen 1 und 2 oder dessen landwirtschaftlich brauchbare Salze auf die Pflanzen und/oder ihren Lebensraum einwirken läßt.

## Claims

**1.** An N-((6-trifluoromethylpyrimidin-2-yl)-aminocarbonyl)-2-carboalkoxybenzenesulfonamide of the general formula Ia

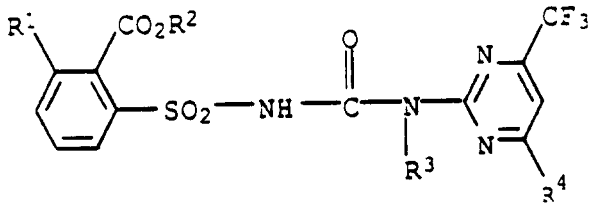

Ia

where $R^1$ is hydrogen or halogen, $R^2$ is hydrogen, $C_1$-$C_5$-alkyl, $C_3$- or $C_4$-alkenyl, $C_3$- or $C_4$-alkynyl, $C_1$-$C_5$-haloalkyl, $C_3$-$C_5$-alkoxyalkyl, $C_3$-$C_5$-haloalkoxyalkyl or $C_5$- or $C_6$-cycloalkyl, $R^3$ is hydrogen, $C_1$- or $C_2$-alkyl, allyl, propargyl or $C_1$-$C_5$-alkoxy and $R^4$ is halogen, $C_1$- or $C_2$-alkyl, $C_1$-$C_3$-alkylthio or $C_1$-$C_3$-alkylamino, and $R^3$ is not hydrogen or $C_1$ or $C_2$-alkyl when $R^4$ is $C_1$- or $C_2$-alkyl, and their environmentally compatible salts.

**2.** An N-((6-trifluoromethylpyrimidin-2-yl)-aminocarbonyl)-2-carboalkoxybenzenesulfonamide of the general formula Ib

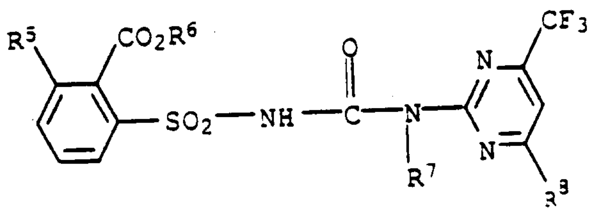

Ib

where $R^5$ is hydrogen, fluorine or chlorine, $R^6$ is $C_1$-$C_3$-alkyl, $R^7$ is hydrogen or methyl, and $R^8$ is hydrogen or $C_1$- or $C_2$-alkoxy.

**3.** An N-((6-trifluoromethylpyrimidin-2-yl)-aminocarbonyl)-2-carboalkoxybenzenesulfonamide of the formula Ia or Ib as claimed in claims 1 and 2, where $R^2$ and $R^6$, respectively, are methyl.

**4.** A process for preparing an N-((6-trifluoromethylpyrimidin-2-yl)-aminocarbonyl)-2-carboalkoxybenzenesulfonamide of the formula Ia or Ib as claimed in claims 1 and 2, wherein a corresponding 2-(isocyanatosulfonyl)-benzoate of the formula IIa or IIb

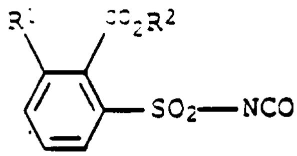

IIa

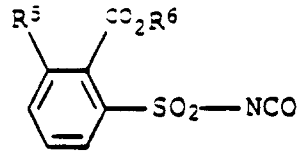

IIb

is reacted in conventional manner with an amine of the formula IIIa or IIIb

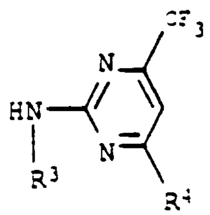

IIIa

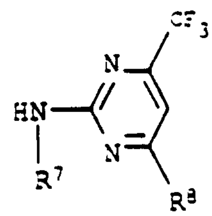

IIIb

**5.** A process for preparing an N-((6-trifluoromethylpyrimidin-2-yl)-aminocarbonyl)-2-carboalkoxybenzenesulfonamide of the formula Ia or Ib as claimed in claims 1 and 2, wherein a corresponding sulfonamide of the formula IVa or IVb

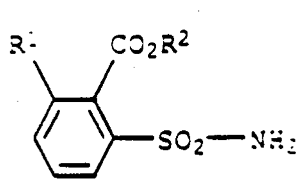

IVa

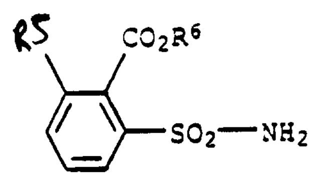

IVb

is reacted in conventional manner with a corresponding phenyl carbamate of the formula Va or Vb

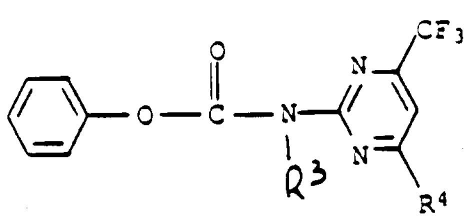

Va

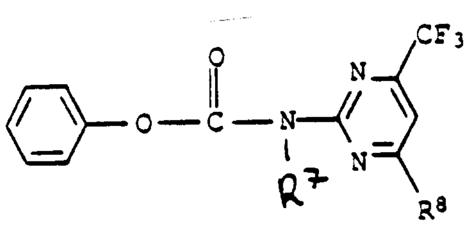

Vb

6. A process for preparing an N-((6-trifluoromethylpyrimidin-2-yl)-aminocarbonyl)-2-carboalkoxybenzenesulfonamide of the formula Ia or Ib as claimed in claims 1 and 2, wherein a corresponding phenylcarbamate of the formula VIa or VIb

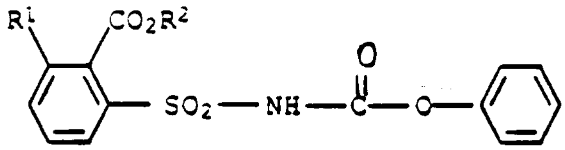

VIa

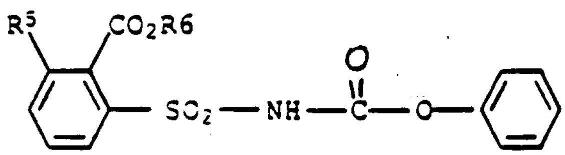

VIb

is reacted in conventional manner with a corresponding amine of the formula IIIa or IIIb as claimed in claim 4.

7. A herbicide containing an N-((6-trifluoromethylpyrimidin-2-yl)-aminocarbonyl)-2-carboalkoxybenzenesulfonamide of the formula Ia or Ib as claimed in claims 1 and 2, or an agriculturally useful salt thereof, in addition to conventional auxiliaries and diluents.

8. A method of controlling undesirable plant growth, wherein an N-((6-trifluoromethylpyrimidin-2-yl)-aminocarbonyl)-2-carboalkoxybenzenesulfonamide of the formula Ia or Ib as claimed in claims 1 and 2, or an agriculturally useful salt thereof, is allowed to act on the plants and/or their habitat.

9. An agent for influencing plant growth, containing, in addition to conventional auxiliaries and diluents, a sulfonylurea of the formula Ia or Ib as claimed in claims 1 and 2, or an agriculturally useful salt thereof.

10. A method of influencing plant growth, wherein an N-((6-trifluoromethylpyrimidin-2-yl)-aminocarbonyl)-2-carboalkoxybenzenesulfonamide of the formula Ia or Ib as claimed in claims 1 and 2, or an agriculturally useful salt thereof, is allowed to act on the plants and/or their habitat.

## Revendications

1. N-((6-trifluorométhylpyrimidine-2-yl)-aminocarbonyl)-2-carboalcoxybenzène-sulfonamides de formule générale Ia

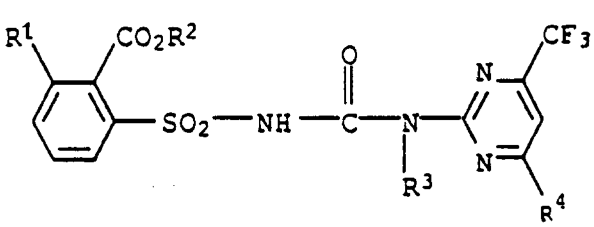

Ia

dans laquelle les symboles ont les significations suivantes :
$R^1$ représente l'hydrogène ou un halogène,
$R^2$ représente l'hydrogène, un groupe alkyle en C1-C5, alcényle en C3-C4, alcynyle en C3-C4, halogénoalkyle en C1-C5, alcoxyalkyle en C3-C5, halogénoalcoxyalkyle en C3-C5 ou cycloalkyle en C5-C6,
$R^3$ représente l'hydrogène, un groupe alkyle en C1-C2, allyle, propargyle ou alcoxy en C1-C5,
$R^4$ représente un atome d'halogène, un groupe alkyle en C1-C2, alkylthio en C1-C3 ou alkylamino en C1-C3,

sous réserve que $R^3$ ne peut représenter l'hydrogène ou un groupe alkyle en C1-C2 lorsque $R^4$ représente un groupe alkyle en C1-C2,
et leurs sels polluants.

2. N-((6-trifluorométhylpyrimidine-2-yl)-aminocarbonyl)-2-carboalcoxybenzène-sulfonamides de formule générale Ib

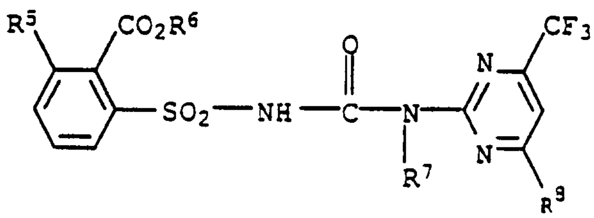

Ib

dans laquelle les symboles ont les significations suivantes :
$R^5$ représente l'hydrogène, le fluor ou le chlore,
$R^6$ représente un groupe alkyle en C1-C3,
$R^7$ représente l'hydrogène ou un groupe méthyle,
$R^8$ représente l'hydrogène ou un groupe alcoxy en C1-C2.

3. N-((6-trifluorométhylpyrimidine-2-yl)-aminocarbonyl)-2-carboalcoxybenzène-sulfonamides de formules Ia et Ib selon les revendications 1 et 2, dans lesquelle $R^2$ et/ou $R^6$ représentent des groupes méthyle.

4. Procédé de préparation des N-((6-trifluorométhylpyrimidine-2-yl)-aminocarbonyl)-2-carboalcoxybenzène-sulfonamides de formules Ia et Ib selon les revendications 1 et 2, caractérisé en ce que l'on fait réagir de manière connue en soi un ester 2-(isocyanatosulfonyl)-benzoique correspondant de formule respective IIa ou IIb

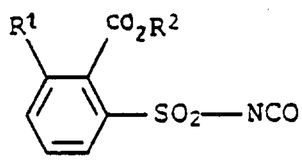

IIa

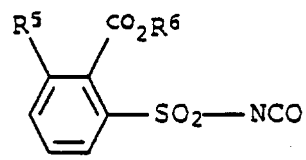

IIb

avec une amine de formule respective IIIa ou IIIb

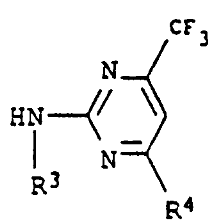

IIIa

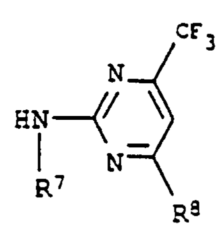

IIIb

5. Procédé de préparation des N-((6-trifluorométhylpyrimidine-2-yl)-aminocarbonyl)-2-carboalcoxybenzène-sulfonamides de formules Ia et Ib selon les revendications 1 et 2, caractérisé en ce que l'on fait réagir de manière connue en soi un sulfonamide correspondant de formule respective IVa et IVb

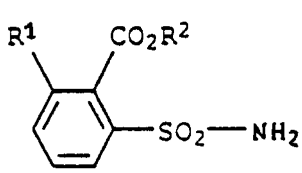

IVa

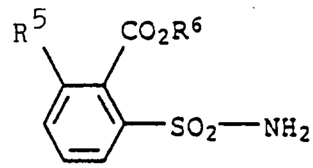

IVb

avec un phénylcarbamate correspondant de formule respective Va ou Vb

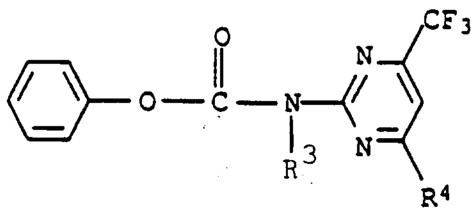

Va

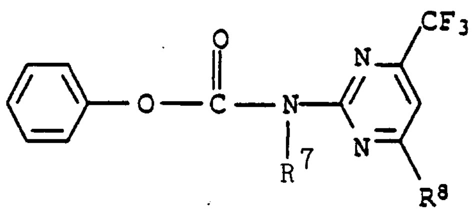

Vb

6. Procédé de préparation des N-((6-trifluorométhylpyrimidine-2-yl)-aminocarbonyl)-2-carboalcoxybenzène-sulfonamides de formules Ia et Ib selon les revendications 1 et 2, caractérisé en ce que l'on fait réagir de manière connue en soi un phénylcarbamate correspondant de formule respective VIa ou VIb

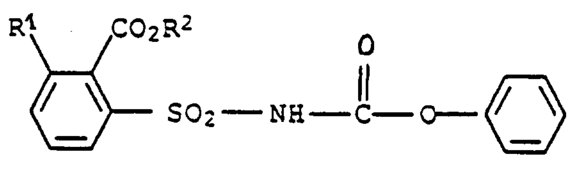

VIa

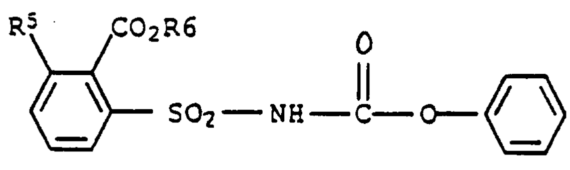

VIb

avec une amine correspondante de formule respective IIa ou IIIb selon la revendication 4.

7. Produit herbicide contenant un N-((6-trifluorométhylpyrimidine-2-yl)-aminocarbonyl)-2-carboalcoxybenzène-sulfonamide de formule Ia ou Ib selon les revendications 1 et 2 ou ses sels acceptables pour les usages agricoles, avec des produits auxiliaires et diluants usuels à cet effet.

8. Procédé pour combattre les croissances de végétaux indésirables, caractérisé en ce que l'on fait réagir sur les végétaux et/ou leur habitat un N-((6-trifluorométhylpyrimidine-2-yl)-aminocarbonyl)-2-carboalco xybenzène-sulfonamide de formule Ia ou Ib selon les revendications 1 et 2 ou ses sels acceptables

pour les usages agricoles.

**9.** Produit pour agir sur la croissance des végétaux, contenant, avec des produits auxiliaires et diluants usuels, une sulfonylurée de formule Ia ou Ib selon les revendications 1 et 2 ou ses sels acceptables pour les usages agricoles.

**10.** Procédé pour agir sur la croissance des végétaux, caractérisé en ce que l'on fait agir sur les végétaux et/ou leur habitat un N-((6-trifluorométhylpyrimidine-2-yl)-aminocarbonyl)-2-carboalcoxybenzène-sulfonamide de formule Ia ou Ib selon les revendications 1 et 2, ou ses sels acceptables pour les usages agricoles.